# EUROPEAN PATENT APPLICATION

(11) **EP 0 712 930 A2**
(43) Date of publication of application: **22.05.1996**
(21) Application number: 95118320.1
(22) Date of filing: 21.11.1995
(51) Int. Cl.: C12N 15/00, A01K 67/027, C07K 14/47

(54) **A transgenic nonhuman animal model for diabetes**

(30) Priority: 21.11.1994 KR 9430676
(71) Applicant: Seo, Jeongsun, Kang-nam-ku, Seoul (KR)
(72) Inventor: Seo, Jeongsun, Kangnam-ku, Seoul (KR); Kim, Soonhee, Songpa-ku, Seoul (KR); Kim, Jongil, Chongno-ku, Seoul (KR)
(74) Representative: VOSSIUS & PARTNER

(57) **Abstract**

Disclosed in this invention is a transgenic nonhuman animal comprising recombinant DNA including a promoter of a gene expressed in pancreatic β-cells and a heat shock protein 70 gene operatively linked downstream to said promoter. Transgenic mice having non-insulin dependent diabetes characterized by a level of 300 mg/dl of glucose in blood were obtained. This transgenic mouse is useful in the fields of research for development of models of diabetes, identification of the mechanism inducing diabetes, development of therapeutic agents and assays for novel medicines.

## Description

The present invention relates to a recombinant DNA comprising a heat shock protein 70 gene operatively linked downstream to a promoter of a gene expressed in pancreatic β-cells, a transgenic nonhuman animal model for diabetes comprising said recombinant DNA and a process for preparing said transgenic nonhuman animal.

Transgenic mice can be created by microinjecting a particular gene fragment into a fertilized egg of a mouse, introducing it into the uterus of another mouse, which will give birth to pups, screening the tail DNA of the offspring 3 weeks after birth, and confirming that the gene is inserted into DNA in the cells of the offspring. A transgenic mouse is useful for detecting functions of a gene in vivo. Improvement of models for many human diseases, various therapeutic agents and bioreactors modifying the body of a mouse to a biofactory are expected fields applicability of a transgenic mouse.

Recently, a novel model according to the transgenic mouse technique as an approach to genetic problems such as cancer has been developed. The success of a c-myc oncogene-inserted transgenic mouse by P. Leder at Harvard University in the U.S.A. has drawn great attention in the field of human leukemia and this technique has become the subject of a patent (U.S. Pat. No. 5,087,571). A male mouse with prostatic disease is disclosed in U.S. Pat. No. 5,175,383, issued to Philip Leder et al., a mouse with depleted mature T-cells is disclosed in U.S. Pat No. 5, 175,384 issued to Paulus J.A. Krimpenfort et al., and a mouse which produces human β-interferon is disclosed in U.S. Pat. No. 5,175,385, issued to Thomas E. Wagner et al.

Insulin, which is secreted from β-cells of pancreatic islets, is a hormone which controls the level of glucose in the blood. Diabetes is a metabolic disease which interferes with metabolism of carbohydrates, lipids and proteins, owing to inadequate insulin supplies, and some forms of diabetes have genetic origins.

Diabetes and complications thereof are the third leading cause of death in the United States, affecting nearly 5% of the population and are very common diseases around the world, and recently diabetes patients have increased in number.

Diabetes can be classified broadly into insulin dependent diabetes (type I) and non-insulin dependent diabetes (type II). Owing to an autoimmune disorder or non-reproduction of damaged β-cells, insulin dependent diabetics need a supply of insulin which is produced outside of the human body and furthermore, juveniles also suffer from insulin dependent diabetes. Non-insulin dependent diabetes is caused by an insufficiency of insulin receptors and decreased insulin binding in the organism although insulin is secreted adequately or excessively from the human body. They occur at a low rate and symptoms thereof are relatively minor. Therefore, insulin dependent diabetes represents only 5 to 10% of all cases, and the rest belong to non-insulin dependent diabetes.

Heat shock protein (Hsp) is an important protein in repairing damage to cells, is expressed in the state of heat shock or stress, at an embryogenic stage as well as during a special step of cell differentiation and the cell cycle (Hunt, C. and Morimoto, R.I., 1985, Proc. Natl. Acad. Sci. USA, 82 (19), 6455-6459).

While the inventor conducted research on the influences of expression of Hsp on differentiation of β cells of pancreatic islets, the inventor found that when a gene containing heat shock protein which was recombined with a human insulin promoter was transferred to a mouse, diabetes developed. Thus, the present invention has been accomplished.

Accordingly, it is an object of this invention to provide a diabetic transgenic nonhuman animal by transferring a gene, which is produced by recombining an Hsp gene with a promoter of a gene expressed in pancreatic β-cells, such as an insulin promoter, into a host animal.

In order to achieve the object, the present invention provides a recombinant DNA, comprising a promoter of a gene expressed in pancreatic β-cells and a heat shock protein 70 gene operatively linked downstream to said promoter, and a transgenic nonhuman animal comprising said recombinant DNA.

Further, the present invention provides a process for preparing a transgenic nonhuman animal comprising the steps of inserting the recombinant DNA comprising the promoter of a gene expressed in pancreatic β-cells and heat shock protein 70 gene operatively linked downstream to said promoter into a fertilized egg, transferring said fertilized egg to a foster mother, and obtaining the offspring of said mother, and a transgenic nonhuman animal prepared by the above process.

The preferred promoter of a gene expressed in pancreatic β-cells is an insulin gene promoter. Further preferred is a human insulin gene promoter. The preferred heat shock protein 70 gene is a human heat shock protein 70 gene.

In this invention, said promoter of a gene expressed in pancreatic β-cells may be prepared by digesting an insulin gene with, for example, EcoRI and BamHI restriction enzymes. Said heat shock protein 70 gene may be prepared by digesting a heat shock protein 70 gene with BamHI and HindIII restriction enzymes. The BamHI site of said insulin promoter can then be ligated to the BamHI site of said heat shock protein 70 gene. Other promoters of genes expressed in pancreatic β-cells may be similarly recombined with Hsp 70 gene using methods well known in the art.

The host animal may be selected, for example, from the group consisting of mouse, pig and chicken. In a preferred embodiment, the host animal is a mouse.

The recombinant DNA is preferably inserted at an embryonic stage of said nonhuman host animal.

In this invention, the process for preparing a transgenic nonhuman animal may further comprise the steps of: mating said offspring with a normal conspecific nonhuman animal to obtain F1 transgenic animals, and mating said F1 transgenic animals among said conspecific F1 nonhuman animals to obtain homozygotic F2 transgenic animals.

Transgenic nonhuman animals expressing non-insulin dependent diabetes and having at least 300 mg/dl of glucose in their blood can be obtained by said method, which is useful in the fields of research for development of models of diabetes, identification of mechanisms inducing diabetes, development of therapeutic agents and assays for novel medicines.

Figure 1 is a schematic representation of a gene which is constructed to express human Hsp 70 under the control of an insulin promoter.

Figure 2 is a schematic diagram showing the procedure for construction of an expression vector (pINS/HSP) containing human heat shock protein 70 gene.

Figure 3 shows the results of analysis by PCR of the DNA taken from the tail of a transgenic mouse.

Figure 4 shows the results of a glucose tolerance test of the transgenic mouse.

Figures 5A and 5B show photographs of pancreatic tissue of a normal mouse and the transgenic mouse, respectively.

The present invention is further explained in more detail with reference to the following examples which does not necessarily limit this invention.

### Example 1: Construction of human heat shock protein gene expression vector

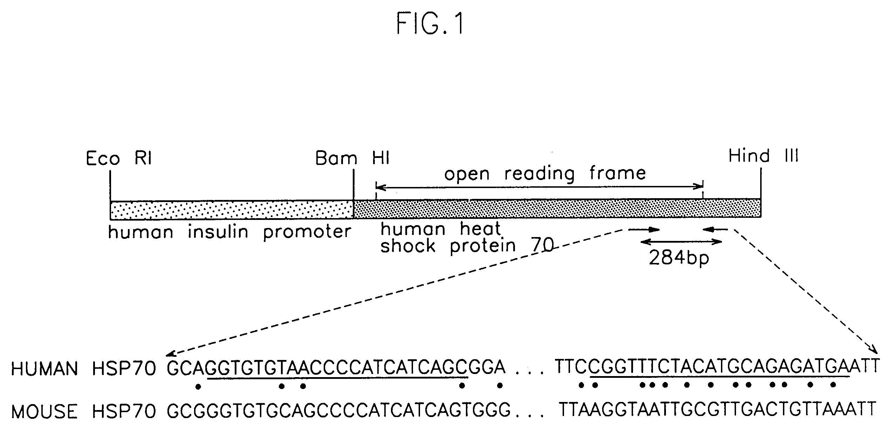

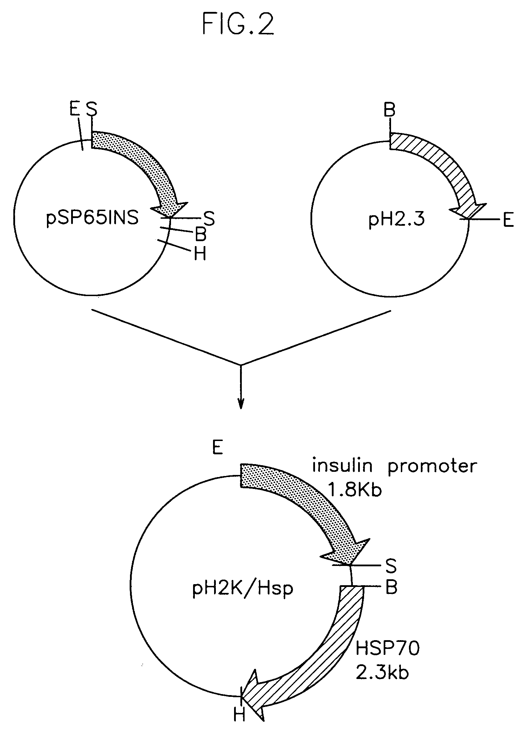

In order to obtain human heat shock protein, pH2.3 (Hunt, C. & Morimoto, R.I., 1985, Proc. Natl. Acad. Sci. 82 (19), 6455-6459) was digested with BamHI and HindIII restriction enzymes to obtain a 2.3 kb DNA fragment. pSP65INS (N. Sarvetnick et al., 1983, Cell, vol. 52, 773-7882) was treated with EcoRI and BamHI restriction enzymes to obtain a 1.8 Kb human insulin promoter. Two fragments were ligated to obtain a 4.1 Kb fragment (Figure 1) and the fragment was cloned into the EcoRI and HindIII restriction enzyme sites of pSP65 purchased from Promega to prepare pINS/Hsp (Figure 2), and then the pINS/Hsp was introduced into HB 101 E. coli to obtain transformed strains. In order to prepare a DNA solution for microinjection, LB medium (1% Bactotrypton, 0.5% Yeast Extract, 1% NaCl) containing ampicillin, was inoculated with the E. coli and cultured at a temperature of 37°C overnight. The culture was collected by centrifugation at 2000g for 10 minutes and dissolved in a solution containing 50 mM glucose, 10 mM Tris-Cl, 1 mM EDTA, 4 mg/ml lysozyme, and allowed to sit for 5 minutes in an ice bath. After 2 volumes of the 0.2 N NaOH containing 1 % SDS solution were cautiously mixed with the resulting solution, the mixture was shaken and allowed to sit at room temperature for 5 minutes. Thereafter, 0.5 volume of 5 M potassium acetate solution was added to the solution, and the obtained mixture was allowed to sit in an ice bath, and then centrifuged at 12,000g for 10 minutes to obtain supernatant. The supernatant was extracted twice or three times with phenol and phenol/chloroform/isoamylalcohol (25:24:1), and plasmid DNA was precipitated by adding 0.1 volume of 3 M sodium acetate and 2 volumes of ethanol to the extract. The precipitated DNA was dissolved in TE buffer solution (10 mM Tris-Cl, pH 8.0/1 mM EDTA, pH 8.0), and CsCl was added to the solution to give 1 g/ml final concentration of CsCl. The solution was centrifuged at 100,000 rpm for 12 hours in a Beckman TL 100 rotor to obtain purified plasmid DNA. The band with the plasmid DNA was separated using a syringe, extracted with n-butanol, and dialysed against TE buffer solution for 24 hours. After plasmid DNA extraction, DNA was digested with HindIII restriction enzyme, and electrophoresed through a 0.8% agarose gel, and DNA was purified by cutting the agarose gel fragment containing the 4.1 Kb DNA. The concentration of the DNA was determined, and then the DNA was diluted with the TE buffer solution containing 10 mM of Tris-Cl, pH 7.5/0.2 mM EDTA, pH 8.0 to a DNA concentration of 4 ng/µl, and stored at a temperature of -20°C to be used for microinjection.

### Example 2: Microinjection of gene of diabetes

A female mouse over 6 weeks old was selected among F1 hybrid strains (C57BL X CBA) which were purchased from Korean Life Engineering Research Institute or a FVB mouse which was purchased from B&K Co. (Great Britain) and superovulated to obtain a great number of fertilized eggs from a few donor mice. The optimum time to microinject DNA into the male pronucleus of a fertilized egg cell is after 1 cell cycle of a fertilized egg of a mouse, and the time depended on the strain from the supplier and the light-dark cycle of the animal cage. The light-dark cycle of the animal cage was set to be turned off at 7.00pm and turned on at 6.00am and microinjection was usually started at 10.30am when using an F1 hybrid strain. One of the fertilized eggs was fixed at a position where the male pronucleus could be seen easily, and 1-2 pl DNA was injected into the male pronucleus with a pipet for injection.

### Example 3: Insertion of microinjected fertilized eggs into the oviduct of a foster mother mouse

The fertilized eggs with DNA inserted were classified into healthy fertilized eggs and dissolved eggs, and then the dissolved eggs were removed. The healthy eggs were transferred to a foster mother mouse on the same day or added to MI6 medium and cultured in an incubator at a temperature of 37°C. A male mouse of the ICR inbred strain which had mated with the foster mother mouse was used. The male mouse underwent a vasectomy, and was used after ascertaining that a pregnancy had not occurred despite the presence of a vaginal plug when it was mated with another female mouse. An ICR female mouse close to the ovulation period was mated with a vasectomised male mouse and if the female had a vaginal plug, the female mouse was used as a foster mother mouse. 0.2 ml of the anaesthetic somopentyl (Pitman-Moor Co., 64.8 mg sodium pentobarbital/ml) diluted 10 times with PBS was inserted into the abdominal cavity, and 20 to 25 fertilized eggs, to which DNA were microinjected, were inserted into both oviducts of the foster mother mouse, and the operated site was sutured.

### Example 4: Breeding of transgenic mice

After confirmation of the insertion of the gene inducing diabetes into a transgenic mouse using DNA taken from the tail and PCR, the transgenic mouse was mated with a normal hybrid mouse (C57BL X CBA) or a FVB inbred mouse of at least postnatal age 6 weeks. Every morning, the vaginal plug was examined for evidence of fertilization. In the case of an abnormal mating, a normal mouse was replaced with the other mouse to be continuously examined. In 3 to 4 weeks after birth (F1), the pup born was analyzed using DNA taken from the tail and PCR. After confirming the transfer of a gene inducing diabetes from the foster female into the pup born, it was named a transgenic mice line. Again, at 6 weeks of age, the F1 transgenic mouse was mated with a normal hybrid mouse (C57BL X CBA) or FVB inbred mouse.

In order to generate a homozygotic transgenic mouse, two mice which were inbred from the foster mother mouse among F1 transgenic mice were mated with each other. In order to detect a homozygotic mouse among F2 transgenic mice, the F2 transgenic mouse was mated with a normal hybrid (C57BL X CBA) mouse or an FVB inbred mouse to generate an F3 mouse. It was then determined whether the F3 mouse had a gene inducing diabetes or not.

### Example 5: Analysis of diabetic transgenic mice

### (1) Extraction of DNA taken from the tail

A DNA polymer was extracted from the tail of a mouse. The extract was analyzed by PCR to determine whether or not the mouse contained the transgene. 1.5-2.0 cm sections of the tail of a mouse which was about 3-4 weeks old, was cut, minced, and added to 500 µl of TE buffer solution containing 50 mM of Tris-Cl, pH 8.0, 50 mM of EDTA, and pH 8.0, 0.5% SDS. Proteinase K was added to the resultant solution to make a final concentration of 200 µg/ml, and the mixture was reacted at 55°C for 9 hours. The reactant was extracted three times with an equal volume of phenol and phenol/chloroform/isoamylalcohol (25:24:1), and was dialysed against 10 mM Tris-Cl, pH 8.0, 1mM EDTA, pH 8.0 for 36 hours (12 hours X 3 times). The DNA concentration was determined using a spectrophotometer and this DNA solution was stored at 4°C.

### (2) Analysis of PCR

77.5 µl of deionized water, 2 µl of sample DNA, 2 µl each of dATP, dGTP, dCTP, dTTP, 10 µl of Taq DNA polymerase buffer (100 mM Tris (pH 8.3), 500 mM KCl, 15 mM MgCl₂, 0.1% gelatin), 2 µl of primer were added to a microfuge tube and mixed. The zone indicated by arrows in Figure 1 represents the primer and the underlined zone represents the base sequence of the primer. Mineral oil was added to the supernatant of the mixture, heated at a temperature of 95°C for 5 minutes to inactivate protease remaining from DNA extraction and to denature the DNA, and the temperature was then changed to 75°C. 0.5 µl of Taq DNA polymerase (2.5 U) was added to the mixture in each tube by passing through the oil layer and then mixing. Thereafter, PCR cycles were carried out as follows: The mixture was denaturated at a temperature of 94°C for 0.5-1 minute, annealed at a temperature of 55°C for 0.5-1 minute, and then extended at a temperature of 72°C for 1 minute. This cycle was repeated 30 times. After completion of the PCR cycles, the final cycle was prolonged for 5 minutes to induce amplified DNA fragments to fully form double strands. The oil layer of the supernatant of the tube was discarded, and then an equal volume of chloroform was added to the resultant mixture. The mixture was centrifuged at 15,000 rpm for 5 minutes. The supernatant was transferred to a new tube, 0.1 times of 3 M NaOAc (pH 5.2) and 2.5 times of 95% EtOH were added to the tube, mixed, and the mixture was cooled at a temperature of -70°C for 15 minutes and then centrifuged at 15,000 rpm, at a temperature of 4°C for 15 minutes to obtain precipitates. The precipitates were rinsed with 70% EtOH, and centrifuged for 5 minutes. The final PCR amplified DNA was dried under vacuum, dissolved in 20 µl of TE buffer solution or distilled water and then 1 to 2 µl of the product was electrophoresed.

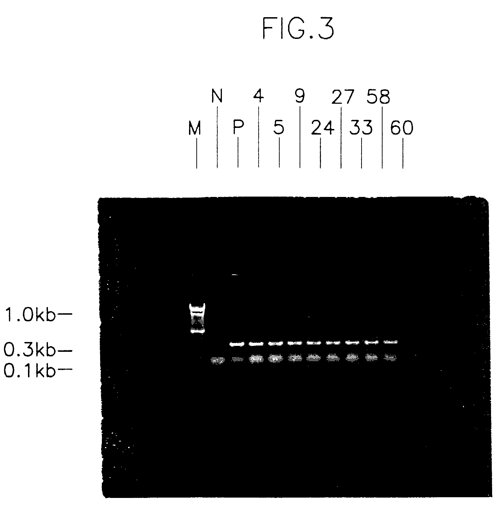

The result of the experiment is shown in Figure 3. In Figure 3 , M represents a 100 base pair ladder, N represents a negative control group and P represents a positive control group. Transgenic mice numbering 4, 5, 9, 24, 33, 58 and 60 showed the same band as that in the positive control group. Therefore, it can be seen that the gene inducing diabetes was transferred into the mice.

### (3) Glucose tolerance test

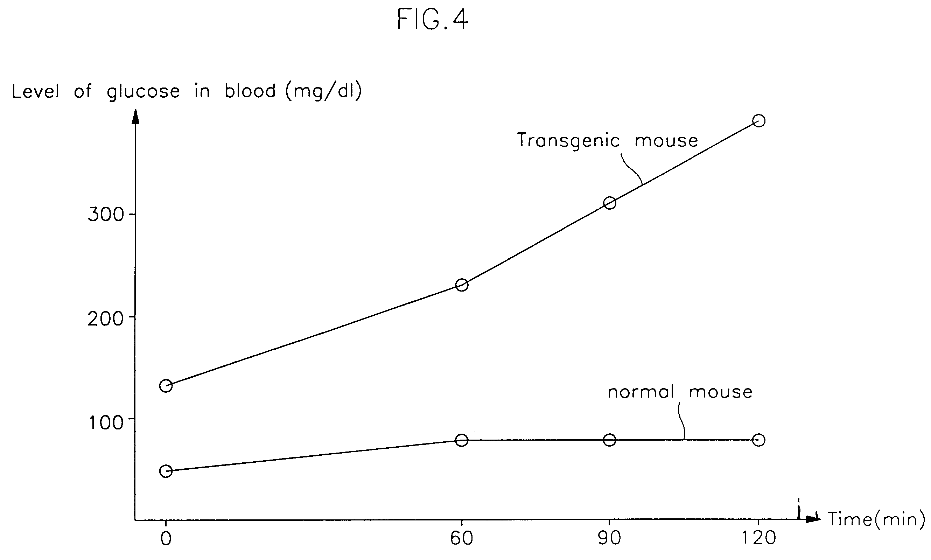

In order to detect whether a transgenic mouse suffered from diabetes or not, 5 mg of sugar was administered to both a normal mouse and a transgenic mouse, and the level of glucose in the blood of the two mice was determined at intervals of 0, 60, 90 and 120 minutes, respectively, as shown in Figure 4. The normal mouse maintained a level of 100 mg/dl or less of blood glucose, however, the level of blood glucose in the transgenic mouse was highly increased. With time, blood glucose increased to a level of 300 mg/dl. Therefore, it can be seen that the transgenic mouse suffered from diabetes.

### (4) Pathological assay of pancreas

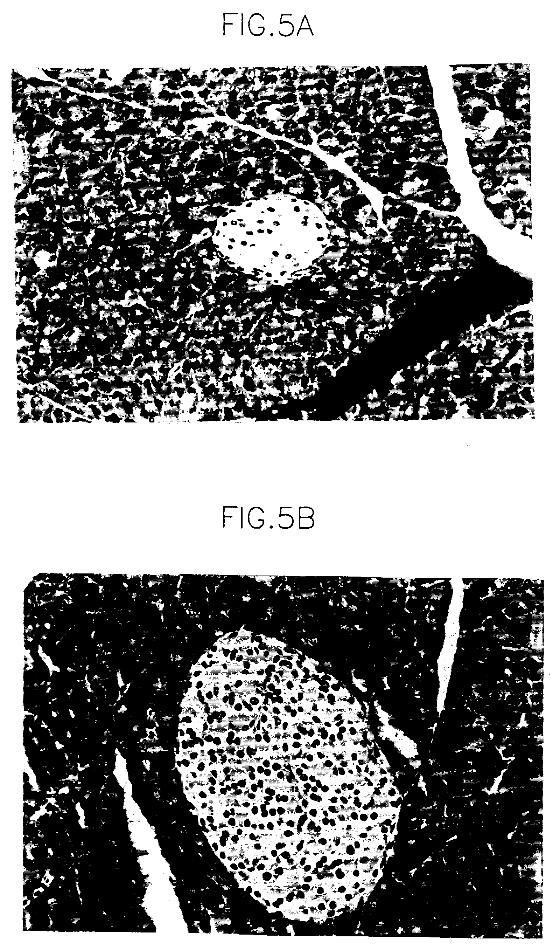

Standard 5-µm sections of pancreas were fixed in 4% paraformaldehyde, embedded in paraffin and stained with haematoxylin in a convention manner. The photographs of the result thereof are shown in Figure 5, wherein Figure 5A is a 200 times enlarged photomicrograph of control pancreas, and Figure 5B is a 200 times enlarged photomicrograph of pancreas from a transgenic mouse. It can be seen that a pancreatic islet of the diabetic transgenic mouse was excessively proliferated compared with the normal pancreatic islet.

As the pancreatic islet was excessively proliferated, it can be assumed that the transgenic mouse suffered from non-insulin dependent diabetes.

## Claims

1. A recombinant DNA comprising:
(a) a promoter of a gene expressed in pancreatic β cells; and
(b) a heat shock protein 70 gene operatively linked downstream to said promoter.

2. The recombinant DNA of claim 1, wherein said promoter of a gene expressed in pancreatic β cells is a promoter of an insulin gene.

3. The recombinant DNA of claim 2, wherein said promoter of an insulin gene is a promoter of a human insulin gene.

4. The recombinant DNA of any one of claims 1 to 3, wherein said heat shock protein 70 gene is a human heat shock protein 70 gene.

5. The recombinant DNA of claim 2, wherein:
(a) said promoter is prepared by digesting an insulin gene with EcoRI and BamHI restriction enzymes;
(b) said heat shock protein 70 gene is prepared by digesting heat shock protein 70 gene with BamHI and HindIII restriction enzymes; and
(c) the BamHI site of said insulin promoter is ligated to the BamHI site of said heat shock protein 70 gene.

6. A transgenic non-human animal comprising the recombinant DNA of any one of claims 1 to 5.

7. The transgenic non-human animal of claim 6, which is a mouse, a pig or a chicken.

8. A process for preparing a transgenic non-human animal comprising the steps of:
(a) inserting the recombinant DNA of any one of claims 1 to 5 into a fertilized egg;
(b) transferring said fertilized egg into a foster mother; and
(c) obtaining the offspring of said foster mother.

9. The process of claim 8, wherein said recombinant DNA is inserted into said fertilized egg at an embryonic stage.

10. The process of claim 8 or 9, further comprising the steps of:
(a) mating said offspring with a normal conspecific non-human animal to obtain an F1 transgenic non-human animal; and
(b) mating said F1 transgenic non-human animal with another F1 transgenic non-human animal to obtain a homozygotic F2 transgenic nonhuman animal.

11. The process of any one of claims 8 to 10, wherein the transgenic non-human animal is a mouse, a pig, or a chicken.

12. A transgenic non-human animal prepared by the process of any one of claims 8 to 11.
